# EUROPÄISCHE PATENTSCHRIFT

(11) **EP 0 995 795 B1**
(45) Veröffentlichungstag und Bekanntmachung des Hinweises auf die Patenterteilung: **07.05.2003**
(21) Anmeldenummer: 99115280.2
(22) Anmeldetag: 02.08.1999
(51) Int. Cl.: C12N 5/10

(54) **Rekombinante Humanzelle für Knochen- und Knorpelzellbildung**
Human recombinant cell for bone or cartilage cell binding
Cellule recombinante humaine pour liaison de cellules osseuses ou cartilagineuses

(30) Priorität: 18.08.1998 DE 19837438
(43) Veröffentlichungstag der Anmeldung: 26.04.2000
(60) Teilanmeldung: 03004303.8
(73) Patentinhaber: Gesellschaft für Biotechnologische Forschung mbH (GBF), 38124 Braunschweig (DE); YISSUM RESEARCH DEVELOPMENT COMPANY OF THE HEBREW UNIVERSITY OF JERUSALEM, Jerusalem 91042 (IL)
(72) Erfinder: Czichos, Stefan, 38124 Braunschweig (DE); Lauber, Jörg, Dr., 38124 Braunschweig (DE); Mayer, Hubert, Dr., 38124 Braunschweig (DE); Gross, Gerhard, Dr., 38124 Braunschweig (DE); Hoffmann, Andrea, 30655 Hannover (DE); Gazit, Dan, Jerusalem, 96920 (IL); Zilberman, Yoram, Jerusalem, 93781 (IL); Pelled, Gadi, Rishon Leziyon, 75424 (IL); Turgeman, Gadi, Jerusalem, 97763 (IL)
(74) Vertreter: Boeters, Hans Dietrich, Dr.

(56) Entgegenhaltungen:
- WO-A-99/11783
- BAMSHAD M ET AL: "Mutations in human TBX3 alter limb, apocrine and genital development in ulnar-mammary syndrome" NATURE GENETICS, Bd. 16, Nr. 3, Juli 1997 (1997-07), Seiten 311-315, XP000864771
- CHAPMAN D L ET AL: "Three neural tubes in mouse embryos with mutations in the T-box gene Tbx6." NATURE, Bd. 391, Nr. 6668, 12. Februar 1998 (1998-02-12), Seiten 695-697, XP002147826
- KAPS C ET AL: "The recombinant expression of bone morphogenetic protein type 1A receptor (Alk3) in mesenchymal progenitors C3H10T1/2 is sufficient for osteo-/chondrogenic development." BIOCHEMICAL SOCIETY TRANSACTIONS, Bd. 26, Nr. 1, Februar 1998 (1998-02), Seiten 27-32, XP000946334 [663rd Meeting of the Biochemical Society; Galway, Ireland; 3-5/09/1997]
- KISPERT ET AL. THE EMBO J. Bd. 14, Nr. 19, 1995, Seiten 4763 - 4772

## Beschreibung

International gibt es trotz intensiver Suche erst einen Transkriptionsfaktor, der auf genetischem Niveau die Bildung knochenformender Osteoblasten auslösen kann. Die Eigenschaften letzteren Faktors wurden kürzlich unter mehreren Namen beschrieben: Osf2, Aml3 oder Cbfa1, wobei es sich immer um den gleichen Faktor handelt. Ein Transkriptionsfaktor für knorpelbildende Chondrozyten existiert zur Zeit nicht.

*Brachyury* (T) ist ein Transkriptionsfaktor, der zu der sog. T-box-Familie gehört. Seine Eigenschaften und die der T-box-Familie wurde kürzlich in mehreren Überblicksartikeln beschrieben (Papaioannou & Silver, 1998; Smith, 1997). *Brachyury* und andere Mitglieder dieser Familie (Tbx1 bis Tbx6) sind in der frühen Embryonalentwicklung aktiv (Herrmann et al., 1990; Kispert et al., 1994; De Angelis et al., 1995; Chapman et al., 1996).

*Brachyury* ist als Transkriptionsfaktor näher charakterisiert, und kürzlich wurde auch die 3D-Proteinstruktur seiner DNA-bindenden Domäne, der T-box, aufgeklärt (Kispert et al., 1995; Müller & Herrmann, 1997). *Brachyury* selbst ist für das Entstehen und die Proliferation undifferenzierter Vorläuferzellen, des Mesoderms, verantwortlich. Andere Mitglieder der T-box-Familie beeinflussen die embryonale Neuralrohr- (Tbx6) (Chapman & Papaioannou, 1998) bzw. Herzentwicklung (Tbx5) (Li et al., 1997; Spranger et al., 1997). Wieder andere Mitglieder dieser Familie greifen voraussichtlich in die embryonale Musterbildung der Gliedmaßen-Entwicklung (Simon et al., 1997) und des Gehirns (T-br1) (Bulfone et al., 1995) ein.

Von diesem Faktor (*Brachyury*) ist seit mehreren Jahren bekannt, daß er in der frühen Embryonalentwicklung eine Rolle spielt und in die Ausbildung des sog. "3. Keimblattes", dem Mesoderm, involviert ist. Die komplette Inaktivierung dieses Faktors *Brachyury* in der Maus (griechisch: kurzer Schwanz; heterozygote Mäuse besitzen einen kurzen Schwanz; ein anderer Name dieses Fakors ist "T", da der Genlocus auch "T-locus" genannt wird) ist in der frühen Embryonalentwicklung lethal und daher für weitere Einflüsse, z. B. bei Knochen- und Knorpelbildung, nicht ohne weiteres analysierbar.

Aufgabe der Erfindung ist es nun, einen Transkriptionsfaktor mit knorpel- und knochenzellbildenden Eigenschaften bzw. eine Humanzelle zur Expression des Faktors vorzusehen.

Diese Aufgabe wird nun durch eine rekombinate Humanzelle für die Knochen- und Knorpelzellbildung von undifferenziertem Mesenchym gelöst, wobei die Humanzelle die Fähigkeit besitzt, Brachyury rekombinant zu exprimieren.

Ferner kann die rekombinante Humanzelle dadurch gekennzeichnet sein, daß sie rekombinant aus einer Zelle aus der folgenden Gruppe gewonnen worden ist:
humane primäre Stromazelle des Knochenmarks,
humane primäre mesenchymale Stammzelle,
humaner primärer artikulärer Chondrozyt,
humaner primärer Chondrozyt aus den Epiphysen von Knochen und primärer humaner Osteoblast.

Ferner kann die rekombinante Humanzelle dadurch gekennzeichnet sein, daß die Humanzelle durch Biopsie gewonnen worden, expandiert und in an sich bekannter Weise rekombinant zur Expression von Brachyury verändert worden ist.

In kritischen Versuchen konnten wir zeigen, daß in einer mesenchymalen Vorläuferzelle (C3H10T½) rekombinant-exprimiertes *Brachyury* die Bildung knochen- und knorpelformender Zellen, den Osteoblasten und Chondrozyten, auslösen kann. Dieser Versuch ist deswegen kritisch, da die normalen parentalen C3H10T½-Zellen stabile Gewebezellen darstellen, die nur auf recht unterschiedliche exogene Signale hin die Möglichkeit der Bildung in vier verschiedenen Bindegewebsformen besitzen: den muskelbildenden Myoblasten, den knochenbildenden Osteoblasten, den knorpelbildenden Chondrozyten und den fettbildenden Adipozyten.

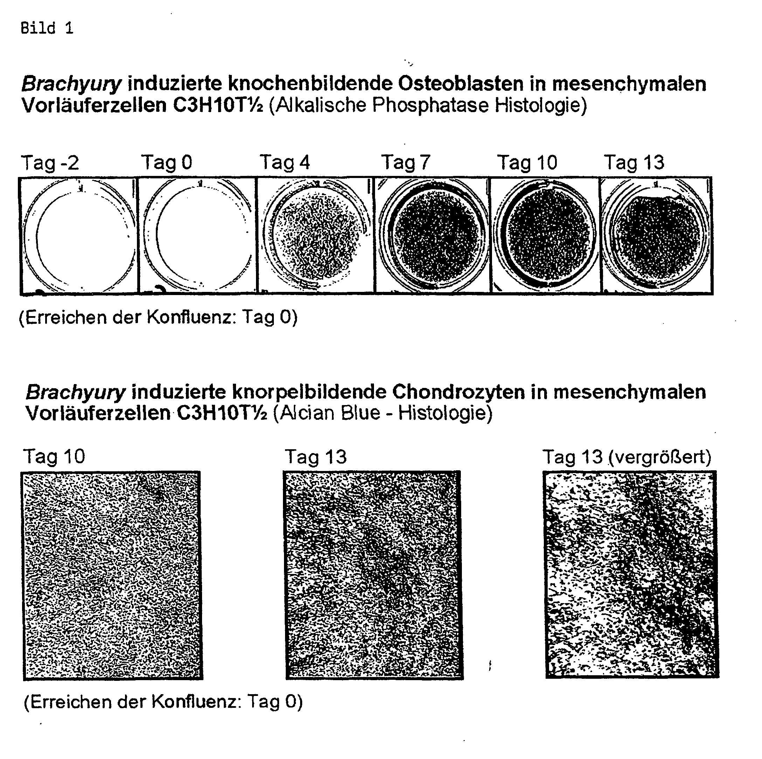

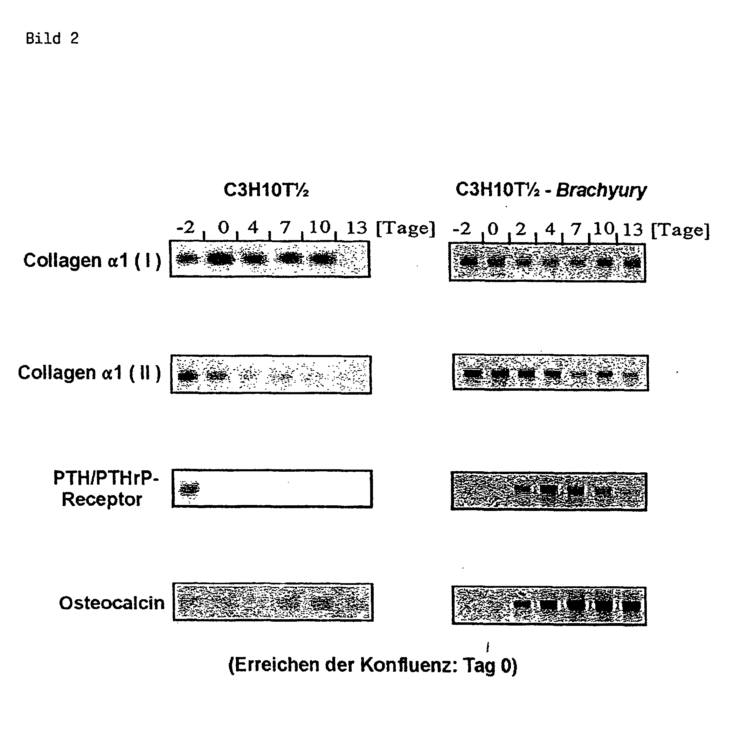

Bilder 1 und 2 dokumentieren, daß *Brachyury* nun in der Lage ist, die Bildung von Osteoblasten und Chondrozyten in diesen C3H10T½-Zellen auszulösen:

### Bild 1:

Histologisch nachweisbare Osteoblasten wurden mit Hilfe der enzymatischen Aktivität von alkalischer Phosphatase, einem Marker-Gen dieser Zellen, nachgewiesen. In der in vitro-Kultur setzt die Reifung der Osteoblasten kurz nach Erreichen der Konfluenz ab Tag 4 ein (das Erreichen der Konfluenz entspricht Tag 0). Eine Woche später sind auch dinstinkte, durch Alcian Blue gefärbte Chondrozyten nachweisbar.

Wesentlich für die Bildung von Chondrozyten und Osteoblasten ist auch der Nachweis der Expression bestimmter Markergene beider Zellformen. Dieser Nachweis wurde hier durch ein Verfahren geführt, das man RT-PCR nennt (Reverse Transkription-Polymerase-Kettenreaktion (PCR). Bei diesem Verfahren wird zuerst aus der gesamten mRNA durch reverse Transkription cDNA hergestellt, danach wird mit Hilfe der PCR und bestimter Primer die cDNA für bestimmte Markergene der Osteoblasten- oder Chondrozytenbildung amplifiziert und dann durch Gelelektrophorese nachgewiesen.

### Bild 2:

Erfindungsgemäß konnte gezeigt werden, daß hochspezifische Markergene für Osteoblastenbildung, wie z. B. das Osteocalcin-Gen und das Gen des PTH/PTHrP-Rezeptors, in *Brachyury*-überexprimierenden Zellen hochreguliert werden. Auch das Collagen (I) zeigt den typisch biphasischen Verlauf der Osteoblastendifferenzierung. Die Chondrozytenbildung wird durch die frühe hohe Expressionsrate von Collagen II bestätigt, außerdem ist auch die Reifung der Chondrozyten wahrscheinlich, da die Bildung von Collagen (II)-mRNA ab Tag 7 der Kultur herunterreguliert wird und dann wahrscheinlich andere Collagene exprimiert werden.

### Referenzen:

Bulfone, A., Smiga, S.M., Shimamura, K., Peterson, A., Puelles, L., and Rubenstein, J.L.R. (1995). *T-brain-1*: A homolog of *Brachyury* whose expression defines molecularly distinct domains within the cerebral cortex. Neuron 15:63-78.
Chapman, D.L., Garvey, N., Hancock, S., Alexiou, M., Agulnik, S.I., Gibson-Brown, J.J., Cebra-Thomas, J., Bollag, R.J., Silver, L.M., and Papaioannou, V.E. (1996). Expression of the T-box family genes, *Tbxl-Tbx5,* during early mouse development. Dev. Dyn. 206:379-390.
Chapman, D.L. and Papaioannou, V.E. (1998). Three neural tubes in mouse embryos with mutations in the T-box gene *Tbx6.* Nature 391:695-697.
De Angelis, M.H., Gründker, C., Herrmann, B.G., Kispert, A., and Kirchner, C. (1995). Promotion of gastrulation by maternal growth factor in cultured rabbit blastocysts. Cell Tissue Res. 282:147-154.
Herrmann, B. G., Labeit, S., Poustka, A., King, T.R., and Lehrach, H. (1990). Cloning of the T gene required in mesoderm formation in the mouse. Nature 343:617-622.
Kispert, A., Herrmann, B.G., Leptin, M., and Reuter, R. (1994). Homologs of the mouse *Brachyury* gene are involved in the specification of posterior terminal structures in *Drosophila, Tribolium,* and *Locusta.* Genes Dev. 8:2137-2150.
Kispert, A., Koschorz, B., and Herrmann, B.G. (1995). The T protein encoded by *Brachyury* is a tissue-specific transcription factor. EMBO J. 14:4763-4772.
Li, Q.Y., Newbury-Ecob, R.A., Terrett, J.A., Wilson, D.I., Curtis, A.R.J., Yi, C.H., Gebuhr, T., Bullen, P.J., Robson, S.C., Strachan, T., Bonnet, D., Lyonnet, S., Young, I.D., Raeburn, J.A., Buckler, A.J., Law, D.J., and Brook, J.D. (1997). Holt-Oram syndrome is caused by mutations in *TBX5*, a member of the *Brachyury (T)* gene family. Nature Genet. 15:21-29.
Müller, C.W. and Herrmann, B.G. (1997). Crystallographic structure of the T domain DNA complex of the *Brachyury* transcription factor. Nature 389:884-888.
Papaioannou, V.E. and Silver, L.M. (1998). The T-box gene family. BioEssays 20:9-19.
Simon, H.G., Kittappa, R., Khan, P.A., Tsilfidis, C., Liversage, R.A., and Oppenheimer, S. (1997). A novel family of T-box genes in urodele amphibian limb development and regeneration: Candidate genes involved in vertebrate forelimb/hindlimb patterning. Development 124:1355-1366.
Smith, J. (1997). *Brachyury* and the T-box genes. Curr. Opin. Genet. Dev. 7:474-480.
Spranger, S., Ulmer, H., Troger, J., Jansen, O., Graf, J., Meinck, H.M., and Spranger, M. (1997). Muscular involvement in the Holt-Oram syndrome. J. Med. Genet. 34:978-981.

## Patentansprüche

1. Rekombinante Humanzelle für die Knochen- und Knorpelzellbildung von undifferenziertem Mesenchym, wobei die Humanzelle die Fähigkeit besitzt, Brachyury rekombinant zu exprimieren und aus einer Zelle aus der folgenden Gruppe gewonnen worden ist:
- humane primäre Stromazelle des Knochenmarks,
- humane primäre mesenchymale Stammzelle,
- humaner primärer artikulärer Chondrozyt,
- humaner primärer Chondrozyt aus den Epiphysen von Knochen und primärer humaner Osteoplast.

2. Rekombinante Humanzelle nach Anspruch 1, **dadurch gekennzeichnet, daß** die Humanzelle durch Biopsie gewonnen worden, expandiert und in an sich bekannter Weise rekombinant zur Expression von Brachyuryn verändert worden ist.

## Claims

1. Recombinant human cell of undifferentiated mesenchyma for bone and cartilage cell formation, the human cell having the ability to express recombinantly Brachyury and being obtained from a cell from the following group:
human primary stroma cell of bone marrow,
human primary mesenchymal stem cell,
human primary articular chondrocyte,
human primary chondrocyte from the epiphyses of bones, and
primary human osteoblast.

2. Recombinant human cell according to claim 1, **characterised in that** the human cell was obtained by biopsy, expanded and altered recombinantly in a manner known per se for the expression of Brachyury.

## Revendications

1. Cellule humaine recombinée pour la formation de tissu osseux et cartilagineux à partir de mésenchyme indifférencié, laquelle cellule, humaine ayant la capacité d'exprimer le facteur Brachyury recombiné et étant choisie dans le groupe comprenant :
les cellules humaines primaires de la moelle osseuse,
les cellules souches primaires mésenchymateuses,
les chondrocytes humains primaires articulaires,
les chondrocytes primaires humains de l'épiphyse d'os et
les ostéoplastes primaires humains.

2. Cellule humaine recombinée selon la revendication 1, **caractérisée par le fait que** la cellule humaine a été obtenue par biopsie, a été multipliée et a été modifiée de manière connue par recombinaison de manière à exprimer le facteur Brachyury.
